# FASCICULE DE BREVET EUROPEEN

(11) **EP 0 754 947 B1**
(45) Date de publication et mention de la délivrance du brevet: **10.10.2001**
(21) Numéro de dépôt: 96401605.9
(22) Date de dépôt: 18.07.1996
(51) Int. Cl.: G01N 33/543, G01N 33/552, C07K 17/14, C12N 11/14

(54) **Dispositif comprenant une substance biologiquement active immobilisée sur un substrat nitrure covalent par un agent de liaison bifonctionnel**
Vorrichtung mit einer durch einen bifunktionellen Kuppler auf einer Nitridsubstrat kovalent gebundene biologisch aktiven Substanz
Device with a biologically active substance covalently immobilised through a bifunctional linker on a nitride substrate

(30) Priorité: 19.07.1995 FR 9508737
(43) Date de publication de la demande: 22.01.1997
(73) Titulaire: C.S.E.M. CENTRE SUISSE D'ELECTRONIQUE ET DE MICROTECHNIQUE SA, 2007 Neuchâtel (CH)
(72) Inventeur: Chai-Gao, Hui, 3012 Bern (CH); Luginbühl, Reto, 3700 Spiez (CH); Sigrist, Hans, 3309 Kernenried (CH); Skinner, Nigel, 2024 St-Aubin (CH); Van Den Vlekkert, Hendrik, 2003 Neuchatel (CH)
(74) Mandataire: Colas, Jean-Pierre

(56) Documents cités:
- EP-A- 0 127 438
- EP-A- 0 350 073
- WO-A-91/16425
- DD-A- 249 632
- DE-C- 4 435 998
- BIOCONJUGATE CHEM, vol. 4, 1993, pages 528-536, XP002014226 COLLIOUD ET AL.: "Oriented and covalent immobilization of target molecules ...."
- JOURNAL OF MOLECULAR CATALYSIS, vol. 43, 1988, pages 293-301, XP000562703 TAMIYA ET AL.: "Characterization of immobilized urease membrane on silicon nitride layer"
- BIOTECHNOL APPL BIOCHEM, vol. 20, 1994, SAN DIEGO, CALIF, pages 251-263, XP002014227 GAO ET AL.: "Photolinker-polymer-mediated immobilization of monoclonal ...."

## Description

L'invention concerne un dispositif comprenant une substance biologiquement active immobilisée sur un substrat nitrure covalent par un agent de liaison bifonctionnel.

La capacité à doser une substance ou mélange de substances dans un milieu donné est d'un grand intérêt dans diverses applications telles que la surveillance de processus, le contrôle de qualité, la détermination de substances polluantes dans l'air et l'eau, le dosage d'analytes dans les fluides physiologiques, le diagnostic de maladies, la compatibilité histologique entre individus, la qualité des aliments, etc..... Les concentrations de substances à mesurer vont de quantités molaires à picomolaires.

On a donc besoin de dispositifs de détection rapides et sensibles à ces fins.

Collioud A. et al., "Oriented and Covalent Immobilization of Target Molecules to Solid Supports: Synthesis and Application of a Light-Activable and Thiol-Reactive Cross-Linking Reagent", Bioconjugate Chem., vol. 4, No 6, 528-536 (1993) révèle l'utilisation d'un agent de liaison bifonctionnel photoactivable permettant de lier une substance biologiquement active à la surface de substrats en polymères organiques.

Il est apparu en particulier qu'il serait avantageux de disposer de dispositifs où la substance biologiquement active serait immobilisée sur un substrat minéral qui serait sensiblement inerte eu égard à une liaison physique (physisorption) avec les molécules de ladite substance et qui serait également intégrable dans des dispositifs électroniques et/ou optiques intégrés. De tels matériaux se trouvent parmi les nitrures covalents tels que le nitrure de bore (BN), les nitrures de titane, les nitrures de phosphore tels que P₃N₅, les nitrures de carbone, les nitrures de silicium et les nitrures de métaux de transition. On préfère notamment le nitrure de silicium en raison de son indice de réfraction, de ses propriétés de guide d'ondes, de ses propriétés de faible absorption optique, et de son inertie chimique, qui le rendent bien adapté à des applications faisant appel à l'optique intégrée.

Dans un domaine technique différent, celui des implants chirurgicaux ou du matériel chirurgical, il existe un besoin constant pour des articles dont les surfaces sont biocompatibilisées, par exemple, afin de minimiser ou éliminer les réactions de rejet ou les réactions adverses du sang (coagulation par exemple) au contact d'implants et d'instruments. Bien que des techniques d'immobilisation de substances biologiquement actives, comme l'héparine par exemple, soient déjà connues, on a toujours besoin d'améliorations dans ce domaine. Il est apparu que des revêtements de nitrures, par exemple de nitrure de titane, appliqués sur des implants ou dispositifs médicaux ou chirurgicaux et sur lesquels serait immobilisée une substance biologiquement active appropriée pourraient s'avérer intéressants.

L'invention vise donc à fournir des dispositifs comportant une substance biologiquement active immobilisée sur un substrat en nitrure covalent.

Plus précisément, l'invention concerne un dispositif comprenant un substrat et au moins une substance biologiquement active liée à au moins une partie de la surface de ce substrat, obtenu par réaction, simultanée ou séquentielle, dudit substrat et de ladite substance avec un agent de liaison bifonctionnel dont l'une des fonctions est photoactivable et génératrice de carbènes et sert à lier l'agent de liaison au substrat minéral, et l'autre fonction sert à lier l'agent de liaison à la substance biologiquement active, caractérisé en ce que ledit substrat est un nitrure minéral covalent.

L'agent de liaison bifonctionnel doit avoir un ou plusieurs groupes fonctionnels pouvant générer des radicaux carbènes par exposition à la lumière (visible ou UV). Des exemples de telles fonctions sont des fonctions diazirines.

L'autre fonction de l'agent de liaison doit être capable de réaction avec la substance biologiquement active, afin de lier l'agent de liaison à cette substance.

Une classe d'agents de liaison préférés est celle des agents homo- ou hétéro-bifonctionnels.

Les agents de liaison homobifonctionnels photoactivables portent à chaque extrémité de la molécule un groupe fonctionnel photosensible, de préférence des fonctions aryldiazirine pour la fonctionnalisation de nitrures covalents. La distance entre les fonctions aryldiazirine photoactivables peut, par exemple, aller de 0,4 à 1,7 nm. Ces agents homobifonctionnels de bas poids moléculaire peuvent être appliqués sur la surface, puis photoactivés, ou bien on peut les mélanger avec la substance biologiquement active et revêtir la surface de ce mélange.

Les agents de liaison hétérobifonctionnels photoactivables portent des groupes fonctionnels différents à chaque extrémité de la molécule et peuvent être de grosseur moléculaire variée. L'un est sélectivement réactif avec la substance biologiquement active tandis que l'autre est un groupe photoactivable, de préférence un groupement aryldiazirine ou alkyldiazirine. Le groupe réactif avec la substance biologiquement active peut être notamment un groupe isothiocyanate, capable de réagir avec des amines primaires, ou une fonction ester de succinimidyloxy, capable de réagir avec des amines primaires, ou un groupe maléimido, dont la double liaison peut réagir par une réaction d'addition avec des thiols. Des esters activés et des anhydrides réagissent facilement avec un groupe hydroxy ou amino dans un solvant organique ou un mélange solvant organique-tampon, si besoin en présence d'un catalyseur de condensation.

L'expression "substance biologiquement active" (SBA en abrégé) doit être comprise dans un sens large et inclut notamment des substances synthétiques ou naturelles de bas poids moléculaire telles que des phospholipides, glycolipides, oligo- et mono-saccharides, aminoacides, peptides, oligonucléotides, médicaments ou métabolites. Ce peut être aussi des macromolécules naturelles ou synthétiques de haut poids moléculaire telles que des protéines, des enzymes, des antigènes, des haptènes, des anticorps, des récepteurs, des protéines de liaison de cellules, des polycarbohydrates, ou des structures biologiques complexes comme des virus, des organites, des cellules, des bactéries ou des sections de tissu. Comme indiqué ci-dessus ces substances peuvent être modifiées par l'agent de liaison avant le revêtement de la surface ou être immobilisées sur la surface après la photoimmobilisation dudit agent sur la surface.

Avantageusement, la SBA peut être immobilisée sous forme d'une couche sensiblement monomoléculaire en réglant convenablement le nombre de molécules de l'agent de liaison liées au substrat par le processus photochimique. Ce mélange peut être obtenu en jouant sur la concentration de l'agent de liaison appliqué au substrat et les conditions de l'exposition au rayonnement (durée, intensité).

L'exposition au rayonnement activant de la fonction génératrice de carbènes peut être effectuée globalement sur une surface du matériau, ou selon un motif de forme désirée en utilisant un masque approprié.

La source de lumière doit émettre des radiations dont la ou les longueurs d'onde se recoupent avec la bande d'absorption de la fonction photoactivable de l'agent de liaison. Dans le cas des aryldiazirines une source de lumière émettant à 350 ± 30 nm s'est avérée satisfaisante. Il est avantageux, en tout cas, d'utiliser un rayonnement de longueur d'onde supérieure à 320 nm pour éviter un effet néfaste éventuel sur la SBA. Des exemples de sources appropriées sont la lumière solaire convenablement filtrée, la lumière polarisée, la lumière d'une source disponible dans le commerce comme le dispositif Stratalinker® , des lampes au mercure haute pression, des lasers, etc...

Une exposition selon un motif peut être obtenue à l'aide d'un masque approprié ou en "écrivant" avec un laser.

Le dispositif de l'invention peut être, à titre d'exemple non limitatif, un biocapteur, un bioréacteur, un implant, un dispositif d'analyse médical ou industriel, ou un dispositif de diagnostic.

Les exemples non limitatifs suivants sont donnés pour illustrer l'invention.

Dans les exemples 1, 4 et 5 on suit le mode opératoire général en trois étapes suivantes :
étape 1 : l'agent de liaison bifonctionnel photoactivable ou le produit de la réaction entre la SBA et l'agent de liaison est appliqué en un revêtement sur une surface propre du substrat.
étape 2 : la surface revêtue est irradiée par une source lumineuse soit globalement, soit selon une image à l'aide d'un masque.
étape 3 : les molécules non immobilisées lors de l'étape 2 sont éliminées par lavage.

Le revêtement peut être effectué par application et séchage, par adsorption physique ou par sédimentation assistée par centrifugation, etc.....

Dans ces exemples on utilise les abréviations suivantes:
- BSA: albumine de sérum bovin
- GOD: glucose oxydase
- HEPES: acide 4-(2-hydroxyéthyl)-pipérazine-1-éthane-sulfonique (fournie par la Société SIGMA)
- MAD: M-{m-[(3-trifluorométhyl)diazirine-3-yl]phényl} -4-maléimidobutyramide (préparé comme décrit par Collioud et al., Bioconjugate Chem. 4, 428-436 (1993).
- PBS: solution saline de tampon phosphate (150 Mm NaCl, 5 Mm tampon phosphate de sodium, pH 7,4)
- SFM: microscopie à force atomique à balayage
- T-BSA: albumine de sérum bovin modifiée par la 3-(trifluorométhyl)-3-(m-isothiocyanatophényl)diazirine (synthétisée comme décrit par Gao et al., Biotech. Appl. Biochem. 20, 251-263 (1994).
- T-GOD: glucose oxydase modifié par la 3-(trifluorométhyl)-3-(m-isothiocyanatophényl)diazirine
- TRIMID: 3-(trifluorométhyl)-3-(m-isothiocyanatophényl)diazirine (préparée comme décrit par Dolder et al., J. Prot. Chem., 9, 407-415 (1990).

### Sources de dérivés réactifs

[¹⁴C]-HCOH, radioactivité spécifiée de 53 mCi mmol⁻¹ New England Nuclear
[³⁵S]-cystéine, activité spécifiée de 20 à 150 Mci mmol⁻¹ AMERSHAM
IgG de souris monoclonal immunologiquement pure PIERCE
Anticorps anti-dinitrophényl (DNP) de souris monoclonal. SIGMA

- Glucose oxydase: SIGMA (β-D-glucose : oxygène 1-oxydoréductase EC 1.1.3.4. Aspergillus Niger)
- Colonne PD10: colonne chromatographique pré-garnie achetée chez Pharmacia
- Photomasques: photomasques en nickel avec des fentes de 300 *µ*m x 15 mm espacées de 500 *µ* m et avec des fentes de 20 *µ*m x 15 mm espacées de 180 *µ*m fournis par TOWNE LABORATORIES INC., SOMERVILLE, N.J., U.S.A.
- Puces de Si, revêtues de nitrure de silicium: Puces de Si (orientation [100], type p) comportant une couche de 100 nm d'oxyde thermique et une couche de 200 nm de nitrure de silicium, produites à l'état prédécoupé (5 x 5 mm) sur demande par l'Institut de Microtechnologie, Université de Neuchâtel, Suisse
- Leviers pour SFM: Des cantilevers pyramidaux et des leviers en nitrure de silicium ont été obtenus de PARK SCIENTIFIC INSTRUMENTS, Sunnyvale CA, USA

### Sources de lumière :

- Stratalinker: Réticulateur U.V. Stratalinker 2400 (Stratagene GmbH, Heidelberg, Allemagne) équipé de 5 bulbes (F158 BL, lumière noire 15W).
- Laser à l'argon: laser équipé d'un miroir à UV (multiraies à 351-363 nm).
- Mesure de l'intensité de la lumière: la dose d'irradiation a été mesurée avec un intensitomètre SUSS, modèle 1000 avec une cellule de détection P à 320 nm.

### EXEMPLE 1

### Liaison de N-{m-[(3-trifluorométhyl)diazirine-3-yl]phényl}-4-maléimidobutyramide (MAD) à du nitrure de silicium.

Des puces de Si revêtues de nitrure de silicium ont été rincées et traitées aux ultrasons dans de l'acétone à trois reprises pendant 15 minutes et une fois dans l'isopropanol pendant 15 minutes pour éliminer le revêtement photosensible protecteur. Ensuite, les puces ont été séchées sous vide pendant 60 minutes (5 mbar, à température ambiante). Du MAD (10 *µ*l, 1 mM dans l'éthanol) a été appliqué à la surface du nitrure de silicium. On a séché les puces sous vide pendant 30 minutes (5 mbar, à température ambiante). Les puces ont été exposées à une lumière activante (source de lumière Stratalinker® , 350 nanomètres, 20 minutes, 0,7 mWcm⁻²) à travers des masques à motif de fentes (300 *µ*m et 20 *µ*m). Après exposition à la lumière les puces ont été lavées et traitées aux ultrasons à trois reprises pendant 10 minutes dans du chloroforme et une fois dans l'éthanol. On a fait alors incuber le substrat pendant deux heures à la température ambiante avec 1 *µ*l de [³⁵S]-cystéine (15 *µ*Ci) dans 80 *µ*l de solvant consistant en tampon phosphate de sodium 0,1 M, pH 6,8 et en éthanol (3/1 v/v). On a rincé la surface modifiée avec de l'éthanol et on l'a traitée aux ultrasons (2 minutes) dans du tampon phosphate de sodium 0,1 M, pH 6,8. Après séchage, un film radiographique (film Hyper, Amersham) a été exposé à la surface traitée pendant 2 jours pour obtenir une image des zones radioactives. On a obtenu un motif à bandes correspondant aux fentes des masques.

### EXEMPLE 2

### Photoimmobilisation selon un motif de T-BSA à du nitrure de silicium.

Des puces en Si revêtues de nitrure de silicium ont été nettoyées et prétraitées comme dans l'Exemple 1 ci-dessus. Du T-BSA (10 *µ*l d'une solution contenant 610 *µ* g de protéine par ml dans un tampon 1,5 mM NaCl, 0,5 mM phosphate de Na, pH 7,4) a été déposé sur la surface de nitrure de silicium et séché sous pression réduite (2 heures, 5 mbar). On a effectué l'irradiation à travers un masque à motif de fentes de 20 *µ*m avec la source de lumière Stratalinker pendant 20 minutes et à une dose d'exposition de 0,7 Mwcm⁻². Ensuite, les puces ont été lavées et traitées aux ultrasons à trois reprises pendant 15 minutes dans un tampon 1,5 mM NaCl, 0,05 mM, phosphate de sodium, pH 7,4 contenant 0,02% de Tween® . Pour l'étude par microscopie à force atomique (AFM), les puces à motifs de 20 *µ*m ont été montées dans la cellule à liquide du microscope à force atomique. Après ajout de PBS, on a laissé le système s'équilibrer pendant 60 minutes avant de former une image par microscopie à force atomique par balayage. Là encore on obtient un motif correspondant à celui du masque à fentes.

### EXEMPLE 3

### Immobilisation de réactifs immunologiques.

Préparation d'immunoglobuline (IgG) de souris radiomarqué au [¹⁴C]. De l'IgG de souris (1 mg dans 500 *µ*l de tampon au phosphate de sodium 100 mM pH 6,8) a été transféré à du tampon HEPES 0,1 M, pH 7,5 par chromatographie sur une colonne chromatographique PD10. On a ajouté ensuite 29,7 *µ*l (1 *µ*mol, 53 *µ*Ci) de [¹⁴C]-formaldéhyde et 65 *µ*mol de cyanoborohydrure de sodium à la protéine recueillie (890 *µ*g dans 1,5 ml de tampon HEPES) et on a agité le mélange réactionnel pendant 4 heures à la température ambiante dans l'obscurité. L'IgG de souris [¹⁴C]-méthylé a été séparé du mélange réactionnel par chromatographie sur du PD10 dans du tampon phosphate de sodium 0,1 M, pH 6,8. On a déterminé la radioactivité spécifique par comptage des scintillations et les concentrations de protéine ont été déterminées en mesurant l'absorption (A^{1%} = 14,0) à 280 nanomètres. Les anticorps modifiés ont été conservés à -20°C.

### Photoimmobilisation des anticorps sur des puces revêtues de nitrure de silicium.

Les anticorps ont été mélangés avec du T-BSA (du BSA pour les échantillons témoins) dans un rapport pondéral de 1/4. Des échantillons contenant 0,25 *µ*g d'anticorps et 1,0 *µ*g de T-BSA ont été ajustés à un volume final de 25 *µ*l (dans du tampon au phosphate de sodium 0,01 M, pH 6,8) et appliqués à la surface de puces de 5 x 5 mm. Les puces ont été placées dans les puits d'une plaque à multipuits Falcon. Après séchage sous pression réduite (2 mbar) à température ambiante les puces revêtues ont été irradiées pendant 20 minutes. Un dispositif de réticulation par ultraviolets Stratalinker 2400 (Stratagene GmbH, Heidelberg) équipé de 5 bulbes (F158 BL, lumière noire 15W) a été utilisé pour l'activation du photomarqueur. Les échantillons ont été placés à 4 cm de distance de la source de lumière et exposés à une dose de radiation de 0,7 Mwcm⁻² (intensitomètre Suss modèle 1000 avec une cellule de détection à 320 nanomètres). On a agité les puces au nitrure de silicium perpendiculairement aux bulbes avec une déflexion de 4 cm et une fréquence de 26 cycles par minute. Des échantillons traités de façon identique et non irradiés ont servi de témoin. Les puces modifiées ont été lavées avec du PBS contenant 0,02% de Tween 20 (3 x 500 *µ*l/puce), de l'eau bidistillée (3 x 500 *µ*l/puce). Le dosage des anticorps photoimmobilisés a été effectué avec les anticorps radiomarqués au [¹⁴C]. On a déterminé ainsi qu'environ 20% des anticorps appliqués ont été photoimmobilisés sur la surface en nitrure de silicium.

### EXEMPLE 4

### Couplage covalent de glucose oxydase à du nitrure de silicium

Avant la photoimmobilisation sur des surfaces de nitrure de silicium l'enzyme glucose oxydase (β-D-glucose:oxygène 1-oxidoréductase, EC 1.1.3.4.) a été modifié par du TRIMID. Brièvement du GOD (81 mg) and du β-D-glucose (1,27 g) ont été dissous dans de la triéthylamine à 0,1%, pH 11,4 et le pH final de la solution a été réglé à pH 10,4 avec de la triéthylamine pure. On a ajouté le TRIMID (170 *µ*l, 29 *µ*mol dans du chloroforme) et on a soumis le mélange à un traitement par ultrasons pendant 30 secondes. Après incubation du mélange réactionnel sous agitation pendant 2 heures à 37°C, on a chromatographié le mélange sur du Sephadex G25 dans un tampon 1,5 mM NaCl, 0,05 mM phosphate de sodium, pH 7,4. Les premières fractions éluées ont été rassemblées et analysées en ce qui concerne leur teneur en protéines et leur activité enzymatique. L'activité enzymatique spécifique de la glucose oxydase modifiée par le TRIMID (T-GOD) était de 0,6 à 1 unité par milligramme de protéine. La teneur en photoréactif lié par covalence était de 8 ± 2 moles de TRIMID par mole de GOD. De la T-GOD (25 *µ*l contenant 2 *µ* g de T-GOD dans un tampon 1,5 mM NaCl, 0,05 mM phosphate de sodium, pH 7,4) a été déposée sur des puces revêtues de nitrure de silicium poli et séchée sous pression réduite à la température ambiante. Des échantillons ont été irradiés par la source de lumière Stratalinker pendant 40 minutes. Ensuite les échantillons exposés à la lumière et des échantillons non irradiés ont été lavés par lavage avec du PBS contenant 0,02% de Tween 20 (3 x 500 *µ* l/puce) et de l'eau bidistillée (3 x 500 *µ*l/puce). On a déterminé l'activité de la glucose oxydase retenue sur les puces par la méthode spectrophotométrique décrite par Foulds et al. (1990), Immunoelectrodes in Biosensors. A practical Approach, CASS, A.E.G. éditeur, IRL Press, OXFORD. Le dosage est basé sur une réaction indicatrice de la peroxydase qui mesure le peroxyde d'hydrogène libéré dans la réaction enzymatique. Ce dosage particulier est basé sur le couplage oxydant de peroxyde d'hydrogène avec de la 4-aminophénazone et un phénol, en présence de peroxydase de raifort pour former un chromogène (un colorant du type quinoneimine) avec une absorption maximale à 520 nanomètres. Les activités enzymatiques mesurées étaient les suivantes :

| Echantillon | Activité enzymatique de la GOD (absorption à 520 nm) |
|---|---|
| GOD photoimmobilisé | 0,48 |
| Témoin | 0,003 |

Des études effectuées avec de la T-GOD radiomarquée ont indiqué que 5% de l'enzyme appliquée était retenue sur la surface après photoimmobilisation. Ceci correspond à 100 nanogrammes par 25 mm².

### EXEMPLE 5

### Fonctionnalisation covalente d'une pointe pour microscope de force à balayage avec un anticorps.

Des cantilevers pour microscopie de force à balayage ont été nettoyés par un traitement aux ultrasons dans l'acétone à trois reprises pendant 15 minutes et dans l'isopropanol à une reprise pendant 15 minutes. On a déposé à trois reprises 5 *µ*l de MAD (100 *µ*M dans l'éthanol) sur la pointe cantilever en laissant la pointe sécher à l'air entre les applications. Les échantillons ont été placés dans l'appareil Stratalinker à une distance de 4 cm de la source de lumière et exposés pendant 20 minutes à une dose d'irradiation de 0,7 mWcm⁻². En variante on a activé la pointe cantilever pendant 60 secondes avec un laser à l'argon (354 nm) focalisé (diamètre du faisceau 50 *µ*m) avec une dose d'irradiation de 130 mWcm⁻². Les pointes cantilevers photoactivées ont été rincées et traitées aux ultrasons à trois reprises pendant 1 minute dans de l'éthanol. Les pointes cantilevers ont été ensuite trempées dans une solution contenant des fragments F(ab') fraîchement préparée (100 *µ*l, 51 *µ*g de protéine) dans de l'acétate de sodium 0,1 M, du chlorure de sodium 0,5 M, et de l'EDTA 1 mM, pH 5,0 et on a laissé incubé pendant 12 heures à 37°C. Pour démontrer la liaison de l'anticorps à la pointe, on a utilisé des fragments F(ab')₂ marqués à l'isothiocyanate de fluorescéine d'un anticorps de souris. Les fragments F(ab')₂ marqués à la fluorescéine ont été traités au dithiothréitol. On a éliminé l'agent réducteur par chromatographie sur colonne PD10 et on a thermochimiquement immobilisé les fragments F(ab') sur des pointes fonctionnalisées par de la MAD. Ensuite on a rincé les pointes cantilevers et on les a traitées aux ultrasons dans un tampon phosphate de sodium 0,1 M, pH 6,0 et on les a laissé s'équilibrer dans ce tampon pendant 2 heures. La liaison des fragments d'anticorps à la pointe par la MAD a été visualisée par microscopie à fluorescence.

## Revendications

1. Dispositif comprenant un substrat et au moins une substance biologiquement active liée à au moins une partie de la surface de ce substrat, obtenu par réaction simultanée ou séquentielle dudit substrat et de ladite substance avec un agent de liaison bifonctionnel dont l'une des fonctions est photoactivable et génératrice de carbènes et sert à lier l'agent de liaison au substrat minéral, et l'autre fonction sert à lier l'agent de liaison à la substance biologiquement active, **caractérisé en ce que** ledit substrat est un nitrure minéral covalent.

2. Dispositif selon la revendication 1, **caractérisé en ce que** ledit substrat est du nitrure de silicium.

3. Dispositif selon la revendication 2, **caractérisé en ce que** le nitrure de silicium est sous forme d'une couche revêtant une puce ou tranche de silicium semi-conducteur.

4. Dispositif selon la revendication 1, 2 ou 3, **caractérisé en ce que** la fonction photoactivable de l'agent de liaison est une fonction diazirine génératrice de carbènes.

5. Dispositif selon l'une quelconque des revendications 1 à 4, **caractérisé en ce que** la réaction de la fonction génératrice de carbènes est activée par une exposition à de la lumière à travers un masque à motif déterminé de manière que la substance biologiquement active soit liée au substrat selon un motif de forme correspondant à celle du motif du masque.

6. Dispositif selon l'une quelconque des revendications 1 à 5, **caractérisé en ce que** la substance biologiquement active liée forme une couche sensiblement monomoléculaire.

7. Dispositif selon l'une quelconque des revendications 1 à 6, **caractérisé en ce que** la substance biologiquement active est choisie parmi les suivantes : ligands, antigènes, haptènes, anticorps, récepteurs, enzymes, organites, cellules, virus, bactéries, tissus, protéines, glycoprotéines, phospholipides, glycolipides, oligo- et mono-saccharides, polycarbohydrates, protéines de liaison de cellule, aminoacides, peptides, oligonucléotides, médicaments et métabolites.

8. Dispositif selon l'une quelconque des revendications 1 à 7, **caractérisé en ce qu'**il est un biocapteur, un bioréacteur, un implant, un dispositif d'analyse médical ou industriel, ou un dispositif de diagnostic.

## Patentansprüche

1. Vorrichtung mit einem Substrat und mindestens einer biologisch aktiven Substanz, die mit mindestens einem Teil der Oberfläche dieses Substrates verbunden ist, erhalten durch gleichzeitige oder sequentielle Reaktion des Substrates und der Substanz mit einem bifunktionellen Bindemittel, dessen eine Funktion fotoaktivierbar und Carbene erzeugend ist und zum Verbinden des Bindemittels mit dem mineralischen Substrat dient und dessen andere Funktion zum Verbinden des Bindemittels mit der biologisch aktiven Substanz dient, **dadurch gekennzeichnet, dass** das Substrat ein covalentes mineralisches Nitrid ist.

2. Vorrichtung nach Anspruch 1, **dadurch gekennzeichnet, dass** das Substrat aus Siliziumnitrid besteht.

3. Vorrichtung nach Anspruch 2, **dadurch gekennzeichnet, dass** das Siliziumnitrid die Form einer Schicht hat, die einen Silizium-Halbleiter-Chip oder -Wafer bedeckt.

4. Vorrichtung nach Anspruch 1, 2 oder 3, **dadurch gekennzeichnet, dass** die fotoaktivierbare Funktion des Bindemittels eine Carbene erzeugende Diazirinfunktion ist.

5. Vorrichtung nach einem der Ansprüche 1 bis 4, **dadurch gekennzeichnet, dass** die Reaktion der Carbene erzeugenden Funktion mittels einer Belichtung durch eine Maske eines vorgegebenen Musters so aktiviert wird, dass die biologisch aktive Substanz mit dem Substrat nach einem Muster entsprechend dem der Maske verbunden wird.

6. Vorrichtung nach einem der Ansprüche 1 bis 5, **dadurch gekennzeichnet, dass** die verbundene biologisch aktive Substranz eine im wesentlichen monomolekulare Schicht bildet

7. Vorrichtung nach einem der Ansprüche 1 bis 6, **dadurch gekennzeichnet, dass** die biologisch aktive Substanz ausgewählt wird aus den folgenden Substanzen: Liganden, Antigenen, Habtenen, Antikörpern, Rezeptoren, Enzymen, Organellen, Zellen, Viren, Bakterien, Geweben, Proteinen, Glycoproteinen, Phospholipiden, Glycolipiden, Olygo- und Mono-Sachariden, Polycarbohydraten, Zellbindungsproteinen, Aminosäuren, Peptiden, Olygonucleotiden, Arzneimitteln und Metaboliten.

8. Vorrichtung nach einem der Ansprüche 1 bis 7, **dadurch gekennzeichnet, dass** sie ein Biosensor, ein Bioreaktor, ein Implantat, eine medizinische oder industrielle Analysevorrichtung oder eine Diagnosevorrichtung ist.

## Claims

1. A device including a substrate and at least one biologically active substance bound to at least a part of the surface of this substrate, which is obtained by simultaneous or sequential reaction of said substrate and of said substance with a bifunctional coupling agent in which one of the functional groups is capable of being photoactivated and generates carbenes and is used to bind the coupling agent to the inorganic substrate, and the other functional group is used to bind the coupling agent to the biologically active substance, in which said substrate is a covalent inorganic nitride.

2. The device of claim 1, wherein said substrate is silicon nitride.

3. The device of claim 2, wherein the silicon nitride is in the form of a layer coating a chip or wafer of semiconductor silicon.

4. The device of anyone of claims 1 to 3, wherein the functional group of the coupling agent which is capable of being photoactivated is a carbene-generating diazirin functional group.

5. The device of anyone of claims 1 to 4, wherein the reaction of the carbene-generating functional group is activated by exposure to light through a mask having a determined pattern so that the biologically active substance is bound to the substrate according to a pattern of a shape corresponding to that of the mask pattern.

6. The device of anyone of claims 1 to 5, wherein the bound biologically active substance forms a substantially monomolecular layer.

7. The device of anyone of claims 1 to 6, wherein the biologically active substance is selected from the group consisting of ligands, antigens, haptens, antibodies, receptors, enzymes, organelles, cells, viruses, bacteria, tissues, proteins, glycoproteins, phospholipids, glycolipids, oligo- and monosaccharides, polycarbohydrates, cell binding proteins, amino acids, peptides, oligonucleotides, medications and metabolites.

8. The device of anyone of claims 1 to 7, which is a biosensor, a bioreactor, an implant, a device for medical or industrial analysis or a device for diagnosis.
